(19) **Europäisches Patentamt** **European Patent Office** **Office européen des brevets**

(11) **EP 1 624 800 B1**

(12) **EUROPEAN PATENT SPECIFICATION**

(45) Date of publication and mention of the grant of the patent:
**03.09.2008 Bulletin 2008/36**

(51) Int Cl.:
*A61B 5/0408* (2006.01)    *G01B 7/16* (2006.01)
*A61N 1/04* (2006.01)    *A61B 5/103* (2006.01)
*A41D 1/00* (2006.01)    *A41D 13/12* (2006.01)

(21) Application number: **04733854.6**

(22) Date of filing: **19.05.2004**

(86) International application number:
**PCT/GB2004/002192**

(87) International publication number:
**WO 2004/100784 (25.11.2004 Gazette 2004/48)**

(54) **KNITTED TRANSDUCER DEVICES**

GESTRICKTE WANDLER

DISPOSITIFS TRANSDUCTEURS TRICOTES

(84) Designated Contracting States:
**AT BE BG CH CY CZ DE DK EE ES FI FR GB GR HU IE IT LI LU MC NL PL PT RO SE SI SK TR**

(30) Priority: **19.05.2003 GB 0311320**

(43) Date of publication of application:
**15.02.2006 Bulletin 2006/07**

(73) Proprietor: **Smartlife Technology Limited Congleton Cheshire CW12 1BD (GB)**

(72) Inventors:
- **DIAS, Tilak**
  **Stockport, Cheshire SK4 2EE (GB)**
- **BEATTIE, Paul Charles William**
  **Marple,**
  **Stockport,**
  **Cheshire SK6 6NY (GB)**
- **COOKE, William**
  **Congleton,**
  **Cheshire CW12 4AF (GB)**
- **WIJESIRIWARDANA, Ravindra**
  **Manchester,**
  **Lancashire M15 6AA (GB)**
- **MITCHAM, Kim**
  **Leicester,**
  **Leicestershire LE3 8QB (GB)**

- **MUKHOPADHYAY, Samir**
  **Claremont,**
  **7700 Cape Town (ZA)**
- **HURLEY, William**
  **Edgeley,**
  **Stockport,**
  **Cheshire SK3 9QX (GB)**

(74) Representative: **Hitchcock, Esmond Antony Marks & Clerk 90 Long Acre London WC2E 9RA (GB)**

(56) References cited:
WO-A-01/02052      WO-A-02/40091
WO-A-03/094717      GB-A- 2 116 725
US-B1- 6 341 504

- FARRINGDON J. ET AL: 'Wearable sensor badge and sensor jacket for context awareness' WEARABLE COMPUTERS, 1999. DIGEST OF PAPERS. THE THIRD INTERNATIONAL SYMPOSIUM ON SAN FRANCISCO, CA, USA 18-19 OCT. 1999, LOS ALAMITOS, CA,USA,IEEE COMPUT. SOC, US 18 October 1999, pages 107 - 113, XP010360090

**Description**

This invention relates to knitted transducer devices and to garments incorporating same.

There are numerous applications in which it is desirable to position sensors on or in the vicinity of a human body. Examples include ECG (electro cardiogram), blood pressure, and temperature measurements, and monitoring of other vital signs. Strain gauges can be used to monitor the expansion of the chest. It is time consuming to apply such sensors, and frequently skilled personnel are required to perform such a task, particularly if it is important to ensure that the sensors are orientated in a defined manner with respect to each other. Furthermore, it is generally only possible to make measurements whilst the subject is present at a defined facility, such as a medical institution. Furtherstill, conventional methods of attaching sensors to a subject can cause discomforture for the subject. Fabric based sensors, including knitted sensors, are disclosed in International Patent Publications WO 02/40091 and WO 03/094717. However, there are limitations to the fabric sensors described therein. Furthermore, in both cases, the manner in which the sensors are coupled to the external detection system is rather cumbersome, requiring a number of manufacturing/assemblage steps. Other sensors are disclosed in US6341504, WO 01/02052 and GB2116725.

The present invention overcomes the above described problems, and provides new classes of flexible transducers which are convenient to produce and may be easily incorporated into wearable garments. The invention is defined by the features of claim 1.

Such transducer devices are flexible, structurally strong and convenient to manufacture and use. Numerous types of devices, such as transducers for strain measurement, proximity detection, and temperature measurement, and knitted microphones and antennae, are provided by the invention.

The first and last courses of the transduction zone are knitted with electrically conductive fibres which act as connecting leads for the knitted transducer device. This design is preferred since it enables the knitted transducer device to be knitted in a single operation using knitting machines such as flat bed knitting machines. Furthermore, the knitted transducer device can be constructed as part of a greater knitted structure such as a garment in a single knitting operation. The connecting leads (used to power the device and transmit a detection signal therefrom) can also be easily incorporated into the structure within a single knitting operation.

The transduction zone may be knitted with combinations of binding elements selected from the group comprising stitches, tuck loops and floats.

In some embodiments, the electrically conductive fibres comprise elastomeric conductive yarn. In such embodiments, there is little redistribution of fibres when the device is flexed, providing different response characteristics to transducers knitted with non-elastomeric conductive yam such as metallic yarn. It is an advantage that there is no or minimal residual strain when the device is knitted with elastomeric conductive yarn. This is because, when a strain on the transducer device is removed, the device can return to a repeatable configuration.

The transduction zone may be knitted with a plurality of types of electrically conductive fibres, each type having a different resistivity.

Examples of electrically conductive fibres are polymeric and metallic fibres. Examples of metallic fibres are steel fibre and copper fibre.

The device may be a resistive knitted transducer device in which deformation of the knitted structure results in a variation of the electrical resistance of the transduction zone. The device may be operable as a strain gauge, in which device:

the transduction zone is knitted with electrically conductive fibres and non-conductive fibres; and
the electrically conductive fibres in the transduction zone extend in a common direction.

The electrically conductive fibres may extend in the course direction of the transduction zone.

The electrically conductive fibres may be incorporated into the transduction zone as laid in fibres, tucks and floats.

The resistance device may be a resistive displacement knitted transducer device in which displacement of the knitted structure from a relaxed configuration results in a variation of the electrical resistance of the transduction zone which is functionally related to the displacement. The transduction zone may comprise:

a transducing section formed from knitting together electrically conductive fibres; and
a plurality of electrical contacts in electrical connection with the transducing section, the electrical contact comprising knitted electrically conductive fibres of a higher electrical conductivity than the electrical conductivity of the electrically conductive fibres in the transducing zone.

The device may be an inductive knitted transducer device in which deformation or movement of the knitted structure results in a variation of the inductance of the transduction zone. The inductive device may be a substantially cylindrical inductive solenoid knitted transducer device. The transduction zone may be knitted from electrically conductive fibres and non-conductive fibres. The electrically conductive fibres may be disposed on the outside of the solenoid and the non-conductive fibres disposed on the inside of the solenoid.

The device may be a capacitive knitted transducer device in which deformation of the knitted structure results in a variation of the electrical capacitance of the transduction zone. The electrically conductive fibres in the transduction zone may define a plurality of spaced apart electrodes. The electrodes may be concentric, interdigitated or parallel plate electrodes. In the latter instance, the transduction zone may further comprise one or more knitted layers of non-conductive fibres extending between the parallel plate electrodes.

The knitted transducer device may comprise a plurality of knitted layers.

According to a second aspect of the invention there is provided an arrangement comprising a plurality of knitted transducer devices according to the invention.

According to a third aspect of the invention there is provided a detection system comprising:

at least one knitted transducer device according to the invention;
electrical supply means for supplying an electrical signal to a knitted transducer device; and
detection means for monitoring an electrical characteristic of a knitted transducer device.

The detection system may comprise a plurality of knitted transducer devices in which the detection means is adapted to derive information pertaining to the relative spatial orientation of the knitted transducer devices. The detection means may derive information pertaining to the relative orientations of the knitted transducer devices by comparing monitored electrical characteristics of the knitted transducer devices.

Electrical characteristics at a plurality of frequencies may be monitored. Typically, an electrical characteristic is measured as a function of frequency in such embodiments. The electrical characteristics may be monitored at a plurality of frequencies in the range 1 to 1000Hz, preferably 5 to 500Hz. These frequencies are particularly useful for ECG measurements.

Electrical impedance is an example of an electrical characteristic that may be monitored. Other electrical characteristics, such as dc resistance and capacitance might be monitored.

The detection means may produce an ECG from the monitored electrical characteristics.

According to a fourth aspect of the invention there is provided a garment comprising at least one knitted transducer device according to the invention. Such garments have the transducers present in situ in their required positions. Thus the correct alignment of the transducers with the body of a wearer of the garment is automatically achieved once the garment is donned and so the garment can be worn by unskilled operatives without requiring skilled supervision. Data can be transmitted remotely by, eg, telemetry and so the subject does not necessarily have to be in the environs of a medical institution. Garments of the present invention are convenient and robust and can even be washed and reused without attention from skilled personnel. Garments of the invention can be used within a hospital environment, and also in the world of sport to collect data from the patient/person wearing the garment. Advantageously, knitted transducer devices can be integrally incorporated into knitted garments in the manufacturing process. Furthermore, knitted structures of the invention are desirable for use in garments, particularly undergarments, owing to a number of advantageous properties, such as good tensile recovery, superior drapability (providing excellent skin contact) and good breathability properties (provided by the air permeability of knitted structures). The garment may comprise a plurality of knitted layers. It is noted in this regard that a garment such as a vest having a front portion of a single layer and a back portion of a single layer is not, for the purposes of the present invention, regarded as having two layers. Rather, it is considered to be a one layer garment but may be described as a (1+1) layer garment. A garment having m layers in the front portion and n layers in the back portion is described as a (m+n) layer garment. Furthermore, for the avoidance of doubt, it should be noted that in the context of the present invention a folded layer knitted fabric, such as described in WO 03/094717, should be regarded as a single layer, and not a two layer arrangement. The knitted layers may be formed as an integral knitted structure, with a knitted transducer present in a knitted layer as part of the knitted structure.

The garment may comprise a first knitted layer having at least one knitted transducer device, and the second knitted layer may be knitted with electrically conductive fibres which act as connecting leads for the knitted transducer device.

The garment may comprise three or more knitted layers. The garment may comprise:

a first knitted layer having one or more knitted transducer devices;
a second knitted layer; and
a third knitted layer having one or more knitted transducer devices.

The second knitted layer may be adapted to screen electrical signals emanating from or introduced to a knitted transducer device located in the third knitted layer. A knitted transducer device in the third layer may be an inductive knitted transducer device. Advantageously, the garment is seamless.

In practice it is usual to employ at least three knitted transducer devices.

Embodiments of knitted transducer devices, garments incorporating same, detection systems incorporating same, and arrangements comprising a plurality of knitted transducer devices in accordance with the invention will now be described with reference to the accompanying drawings, in which:

Figure 1    shows a plurality of resistive strain gauges;

Figure 2    shows an electrical equivalent circuit of a strain transducer;

Figure 3    shows a knitted resistive displacement transducer;

Figure 4    shows the geometrical path of the electroconductive yarn in the transducer of Figure 3;

Figure 5    shows (a) a stitch and (b) a four resistance equivalent circuit of the stitch;

Figure 6    shows an equivalent resistant mesh circuit;

Figure 7    shows a solenoid transducer;

Figure 8    shows impedance characteristics of the solenoid of Figure 7;

Figure 9    shows (a) a diagrammatic representation and (b) a photograph of an arrangement of three solenoid transducers on a finger;

Figure 10   is a plan view of a capacitive proximity transducer;

Figure 11   is a plan view of a capacitive strain gauge;

Figure 12   shows (a) a perspective view and (b) a cross sectional view of an array of parallel plate capacitive transducers;

Figure 13   is a cross sectional view through a (1 + 3) layer garment worn by a subject;

Figure 14   shows (a) front views of the layers in a (3 + 3) layer garment (b) a cross sectional view and (c) a top view of a (3 + 3) layer garment worn by a subject;

Figure 15   shows (a) a perspective view and (b) an exploded view of a (1 + 3) layer vest;

Figure 16   shows the arrangement of the welts during knitting;

Figure 17   is a knitting sequence for the three layered welt;

Figure 18   shows the knitting and transference sequence for the fashioning of three layers on the right selvedge of the vest;

Figure 19   shows the knitting and transference sequence for the fashioning of the three layers on the left selvedge of the vest;

Figure 20   shows the knitting and transference sequence for the bind off and finish of the straps at the shoulders;

Figure 21   shows the knitting and transference sequence for the fashioning of three layers for the left middle neck opening of the vest, also joining of the front and middle panels;

Figure 22   shows the knitting and transference sequence for the fashioning of the three layers for the right middle neck opening of the vest, also joining of the front and middle panels;

Figure 23   shows the knitting and transference sequence for the completion of the knitting sequence for the main body of the three layer vest;

Figure 24 shows an example layout of transducer devices knitted into the middle layer of the vest;

Figure 25 shows a knitting sequence for an example transducer device.;

Figure 26 shows an electrical equivalent circuit of the skin/electrode interface;

Figure 27 shows (a) the equivalent circuit of a measuring arrangement and (b) the equivalent electrical circuit of an electrode system; and

Figure 28 shows an ECG signal obtained using a knitted electrode.

The present invention provides flexible transducers which may be used in the field of wearable electronics. The invention provides knitted structures from, eg, electro-conductive polymeric, metal and smart fibres that will behave as transducers. These transducers can be fabricated using flat-bed, circular and warp knitting technology.

Various non-limiting classes of transducers are described below. The disclosed methods are not part of the claimed invention.

**Resistive fibre mesh transducers**

The knitted transducers are constructed by using electro conductive yarns. The generic method of construction of the transducers is to knit a pre-determined area of the base knitted structure from the electro conductive yarn. The above area is defined as the Electro Conductive Area (ECA) in the following text. The size, shape and the binding elements (stitches, tuck loops, floats and laid-in-yarns) and their organization in the base knitted structure would determine the overall electrical characteristics of the ECA, and its response to structural deformation(s) of the knitted structure. This variation of the electrical characteristics of the ECA would determine the type of knitted transducer and its function.

One of the measurable electrical properties of the knitted transducers is the electrical resistance and its variation of the ECA. The variation of resistance can be captured using two different approaches. Generally, when a knitted structure is deformed the structural deformations are due to the yarn deformations and/or slippages between the yarn contact areas of stitches (stitches are the basic elements of a knitted structure). The yarn deformations may be due to stretching, bending, twisting and compressing. The first method of capturing the electrical resistance of a knitted strain gauge is by considering only the length variation of the conductive yarn in the knitted structure, which results in a variation of resistance.

The second method is by considering the structural deformations of the stitches in the ECA which will results in a variation in electrical resistance. Under small loads this functions as a potentiometer, and, therefore, we have defined these as displacement transducers. The invention is not limited by these two proposed modes of operation.

**Resistive strain gauge**

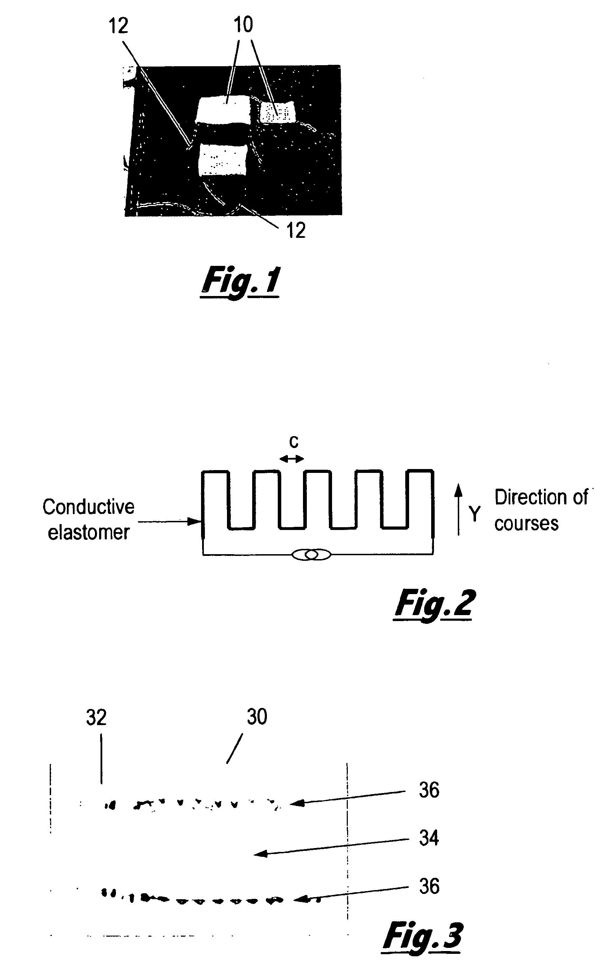

A knitted resistive strain gauge can be made as a single knitted layer using an electro-conductive elastomeric yarn (e. g. carbon filled silicon fibre yarns, typically with a specific resistance of 5-6 KΩ/cm) and non conductive base yarn (which could be an elastomeric yarn). The base knitted structure is formed using the non-conductive yarn and the conductive yarn is laid in the course direction of the base structure in a pre-determined configuration, which could be in the geometrical form of a rectangle, a square, a triangle, a circle or an ellipse. Figure 1 shows a plurality of knitted transducer devices 10 which were fabricated using these principles.

Due to the above configuration it is likely that the variation of resistance will only be due to the stretching of the conductive yarn. If the base structure of the strain transducer is a rib, interlock or a derivative then the conductive elastomer will be incorporated into the knitted structure as a laid in yarn; and the conductive elastomer will be incorporated into a plain or a purl or a derivative in the form of tuck loops and floats. In Figure 1 the structures 10 have been knitted with a white non conductive yarn and with a black laid in conductive elastomer yarn. The black conductive yarn also extends out of the knitted transducer devices 10 to serve as connecting leads 12.

The electrical equivalent circuit of the strain transducer can be modelled by considering the geometrical path of the conductive elastomeric yarn. Since the conductive elastomer yarn is secured within the courses (row of stitches) of the base structure, which has been knitted from the non conductive yarn, the laid in conductive elastomer yarn lies electrically insulated in the base structure, i.e. there are no cross connections in the laid in elastomer yarn. Therefore, the electrical path will be the same as the geometrical path of the laid in conductive elastomer yarn. The equivalent resistance can be calculated, assuming it is electrically powered from the two ends (Figure 2) of the conductive elastomer. When the structure is stretched in the direction of Y the electrical resistance of the electro conductive elastomer yarn would increase with respect to the extension. The resistance of the conductive elastomer will be

$$R = \rho L / A \ldots\ldots\ldots\ldots\ldots\ldots\ldots\ldots\ldots\ldots\ldots\ldots\ldots\ldots\ldots\ldots\ldots\ldots\ldots\ldots(1)$$

where $\rho$ is the resistivity of the conductive elastomer
L is the length of the laid in conductive elastomer and
A is the cross sectional area of the conductive elastomer

**Resistive displacement transducer**

Resistive displacement transducers can be constructed from electro conductive yarn such as polymeric and metallic yarn. The function of the displacement transducer is based on the change of the electrical resistance of the ECA. The polymeric yam is used to produce the ECA to provide the required resistance variation (due to its higher specific resistivity), and the metallic yarn such as stainless steel is used to power the ECA. The deformation of the ECA will result in a change of its electrical resistance. This change will generate an electrical signal if the ECA is electrically powered.

Generally two distinctive structural deformations can be defined for a knitted structure. At the early stage of the deformation of a knitted structure there is a free movement of the yarn in the stitches. The second stage is defined due to the jamming of the yam in the contact areas of the stitches; at this stage the yam can no longer move freely within the structure. The component which contributes more towards the resistance variation in the ECA would depend on the mechanical properties and the surface morphology of the conductive polymeric yam and the load responsible for the deformation of the knitted structure. For small loads the deformation of the stitches would be primarily due to free yam movement, hence the transducer can be used to determine displacement. On the other hand for relatively large loads, i.e. when yam is jammed the deformation of the knitted structure will be due to the yarn deformations (bending and stretching) and the transducer act as a strain gauge.

An example of a knitted displacement transducer 32 is shown in Figure 3. The device 30 comprises a single knitted layer, although it would be possible to provide a multiple layer device instead. The base structure 32 of the transducer is a plain knitted structure. The base structure 32 was knitted with a non conductive yarn, and a rectangular ECA 34 was created by knitting a defined number of wales (n) over a defined number of courses (m) using an electro conductive monofilament yam (1-10K$\Omega$/cm). The ECA 34 was electrically powered using two parallel rails 36 constructed by knitting two courses from a high conductive yarn (stainless steel 1-5$\Omega$/cm).

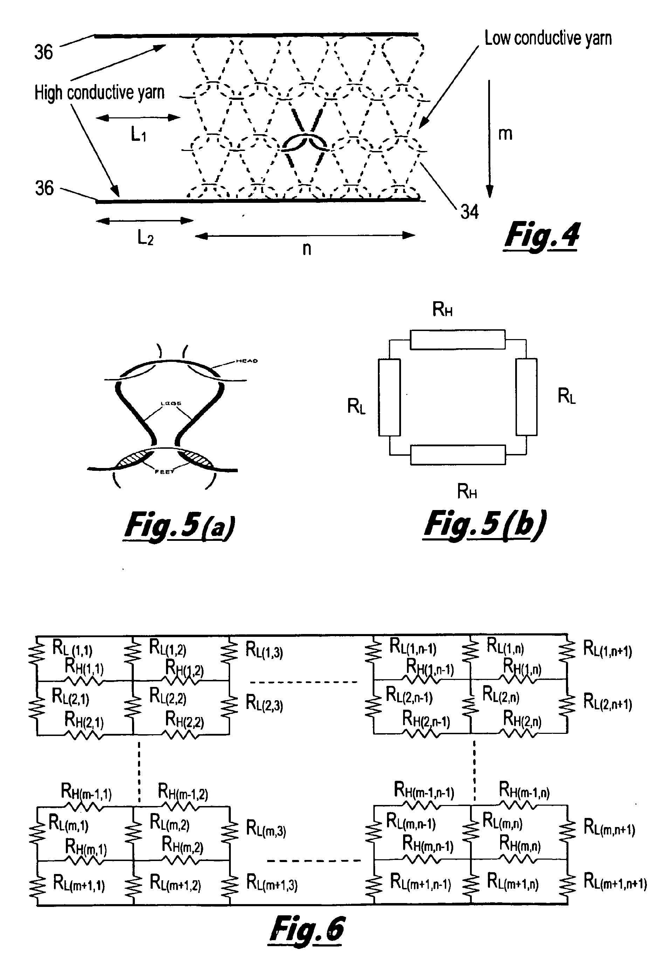

The electrical resistance of the ECA 34 given in Figure 4 will depend on:

- Resistivity of the electro conductive material;
- Number of courses (m) & number of wales (n);
- Geometrical yarn path of the stitches;
- Powering orientation.

In order to calculate the resistance of the ECA its basic building block should be considered, which is a stitch. The plane view of the stitch structure is shown in Figure 4. Any textile structure is created by the physical binding of yarns, and in the case of a knitted structure this is achieved by interconnecting loops formed from yarn, which results in four yarn contact regions. Mechanics of the yam contact regions are very complex, and the behaviour of knitted structure is not yet fully understood, although this does not affect the viability of the device as a transducer. When a knitted structure is in the fully relaxed state the yam contact is more likely to be a line contact; however when a planar force is applied one has to consider the yarn contact as an area contact due to the compressibility of the yarn. Therefore it is reasonable to assume that the yarn contact region as a short circuiting point which then can be considered as a node in an electrical impedance network. A stitch has four contact regions or points. Therefore the DC equivalent circuit of the stitch can be formulated using the resistances of the conductive segments (known as head, legs and feet in knitting terminology) between these four nodes for a stitch. The lengths of the conductive segments are calculated by considering the yam path geometry of the stitch. Therefore, the DC equivalent circuit of a stitch can be defined using four resistances; two equal resistances representing the head and the feet (RH) and two equal resistances representing the two legs (RL). The DC equivalent circuit of a stitch is shown in Figure 5(b). The overall resistance of the ECA is modelled by repeating the four resistance equivalent circuit model of a stitch which constitutes a resistive mesh as shown in Figure 5(b). The total equivalent resistance of the ECA can be calculated for a given powering configuration.

When the ECA is stressed in the direction of the wales, RL is increased and RH is decreased, which is due to the free movement of yam in the contact areas and due to the extension of the yarn segments forming the legs and compression of the yarn segments forming the heads of the stitches. The actual mechanism depends on the structural dynamics. The length of the above yarn segments are calculated by using a geometrical model of the plain knitted structure which demonstrates the deformation of the structure for defined loading conditions. The extension of the length in the yarn segments is then used for the calculation of resistance. For example, for the ECA shown in Figure 4 the powering configuration of the total equivalent circuit is described below. The total resistance of the ECA is calculated using RL and RH values of each unit cell (stitch) by constructing the equivalent resistive mesh (Figure 6).

Mesh theory is applied to calculate the equivalent resistance of the mesh (ECA).

$$V_m = Z_m.I_m \dots\dots\dots\dots\dots\dots\dots\dots(2)$$

$$I_m = Z_{m-1} V_m \dots\dots\dots\dots\dots\dots\dots\dots(3)$$

$$R_{eq}=1/z-1(1,1) \dots\dots\dots\dots\dots\dots\dots\dots(4)$$

Where $V_m$ and $I_m$ are the voltage and current vectors respectively
$Z_m$ is the mesh impedance matrix
z-1(1,1) is the first element of the $Z_m$-1.

When the structure is deformed the new resistance of the ECA and the resistance variation ΔR is calculated:

$$\Delta R = Req-Req1$$

Where Req1 is the initial resistance of the structure under relaxed conditions.

**Inductive fibre mesh transducers**

**Inductive strain gauge**

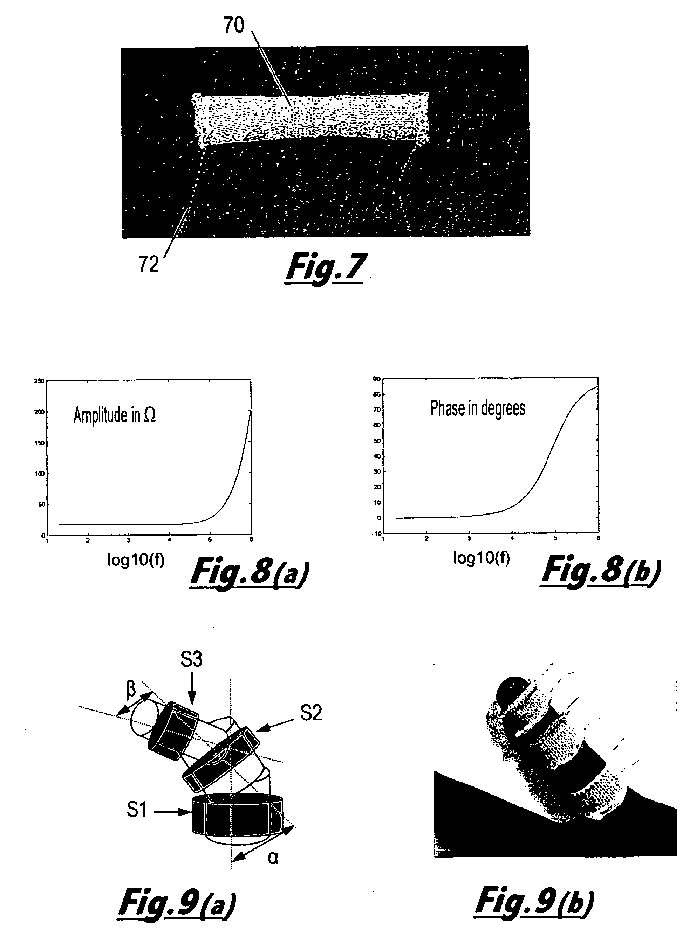

In one embodiment a solenoid is knitted using electro conductive yarn together with a non conductive yarn, preferably an elastic yarn such as Lycra or Spandex in order to create the required elastic properties. The structure can be any one of the basic knitted structure or their derivatives or any combination, knitted in a tubular form. The electro conductive yam can be a polymeric or a metallic yam; however a yam with a very low electrical resistance is preferred. An example of a solenoid 70 made from copper (Cu) wire of gauge 36 is shown in Figure 7. The two yarns (Cu wire and elastomeric yarn) were knitted using plaiting technique, i.e. the yarns were delivered to the knitting needles in such a manner that the conductive cu wire appears on the outside layer and the elastomeric yam appears on the inner layer (side) of the knitted tube (solenoid). The conductive wire extends from the solenoid 70 to act as connecting leads 72 which are connected to suitable means for energising the solenoid 70 and detecting variations in inductive properties of the solenoid 70.

Impedance characteristics were analysed under static and dynamic mechanical loading conditions. The results obtained thereby are shown in Figures 8(a) and 8(b). The electrical characteristics under relaxed state demonstrate a typical high pass filter characteristic. The cut-off frequency was 103 KHz, and, therefore, for better performance the solenoid transducer was driven at 1 MHz to measure strain, i.e. in strain gauge mode.

**Inductive displacement transducers**

The solenoid structure described above can be used as a displacement transducer. However the knitted structure should be made more stable in order to maintain a lower variation of self inductance. Preferably, a higher μr material such as stainless steel yam (which has a μr value 1000 times greater than that of Cu) is used with or instead of the Cu wire to increase the inductance of the solenoid. This improves the transducer resolution. The basic principle is to knit an array of solenoids into a garment (minimum of two) at defined positions. A relatively simple and non-limiting example is given in Figure 9 which shows an arrangement of three solenoids 90, 92, 94 for capturing the gestures of a finger. The use of a greater number of solenoids (or other inductive transducer devices) is within the scope of the invention. S1, S2 and S3 represent the three knitted solenoids 90, 92, 94.

Two methods might be used to measure the angular displacements (α, β). In the first method the mutual impedance variation is measured and in the second method the electromagnetic induction variation is used. The measurements are carried out for a pair of solenoids. For the example shown in Figure 9 the solenoids S1 and S2 were considered as the first pair 1 and the solenoids S2 and S3 as the second pair. The two pairs were energized alternately. The mutual inductance variation of the first pair due to the bending of the finger was used to measure the angular displacement $\alpha$. Similarly the second pair was used to measure the angular displacement $\beta$. The two pairs were energized at 1MHz and the time division multiplexing technique was used to measure the readings of the two solenoid pairs at a frequency of 10 Hz.

In the second method first the solenoid S1 was energized and voltages induced in the solenoids S2 and S3 were measured. Then the solenoid S2 was energized and the voltages induced in solenoids S3 and S1 were measured and finally the solenoid S3 was energized and the voltages induced in solenoids S2 and S1 were measured. The angular positions $\alpha$, $\beta$ were calculated from the data.

The integration of knitted solenoid arrays in to a knitted garment (preferably using the seamless knitting technology) enables the detection (measurement) of elbow and knee movements and knee movements. Similarly, knitting solenoid arrays into a glove during its manufacture enables the detection of the finger movement, thus providing the platform for the creation of a virtual keyboard for PCS, music, games and other niche applications.

**Capacitive fibre mesh transducers**

Capacitive knitted transducer devices can be produced, and it is possible to knit an array of such transducers within a base structure. The base structure can be knitted from a non-conductive yam and electrodes are created within the base structure using an electro conductive yarn, preferably a low resistance yarn such as stainless steel or copper wire. Deformation of the structure results in a displacement of the electrodes and the change of capacitance in the electrodes can be used to measure the mechanical strain, the touch, the displacement and the proximity.

**Capacitive proximity transducers**

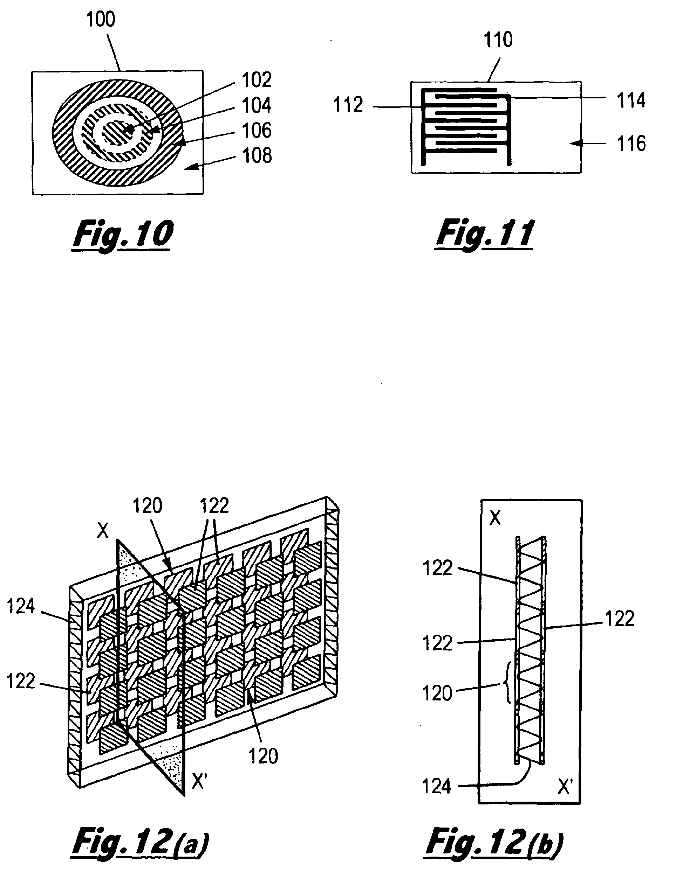

The construction of a touch or proximity transducer 100 is shown in Figure 10. The capacitive proximity transducer 100 is constructed using two knitted layers (multi-layer structure). The transducer comprises two concentric electrodes 102, 104, a compensating electrode 106 and a base knitted structure 108. The electrodes 102, 104, 106 are knitted on to one layer and their connectors are knitted on the second layer (layer underneath the layer with electrodes). The described proximity transducers can also be used as flexible switches in smart garments.

**Knitted capacitive strain gauge**

Figure 11 shows a capacitive strain gauge 110 comprising interdigitated electrodes 112, 114 and a base structure 116 knitted from non-conductive yarn. The strain gauge 110 may be produced as a single knitted layer.

By arranging the electrodes 112, 114 as shown in Figure 11 (defined as a "comb" electrode configuration) the structural deformation of a knitted structure can be measured. The structural deformation may be caused by a mechanical loading of the structure. In order to improve the performance of the strain transducer 110 a non conductive elastomeric yarn can be used to knit the base structure 116.

**Knitted parallel plate capacitor**

A parallel plate capacitive transducer may be constructed by using multi-layer knitted structures (these are also known as spacer structures). A spacer structure consists of two independent planar knitted structures that are interconnected by a monofilament yarn. Spacer structures can be conveniently knitted on modem electronic flat-bed knitting machines by using three different yarns (yarn carriers). An advantage of knitting to fabricate a parallel plate arrangement is the capability of knitting electrodes of defined size, shape and their precision positioning within a ground structure. The electrode plates are constructed by knitting them separately on to the individual planar knitted structures using conductive yarns and the space between the plates is constructed by knitting a high modulus non conductive monofilament yarn. A capacitive array is constructed by knitting the electrode plates according to a predetermined grid on to the individual planar knitted structures, thus isolating the conductive plates. When constructing the transducer(s) an important consideration is to select the yarns of the plate faces to have similar mechanical properties. Advantageously, the whole structure is knitted in one go. Figure 12 shows an array of parallel plate capacitive transducers 120 comprising electrode plates 122. A base layer 124 comprising a non-conductive yarn extends between the electrode plates 122.

## Modelling of Electrical Equivalent Circuit of a Knitted Electrode

When an electrode comes in contact with the skin and electrical interface is formed between them. The interface formed has two components: 1) between the electrode and the electrolyte; and 2) between the electrolyte and the skin. The second component depends primarily on the epidermis layer which consists of three sub layers. This layer is constantly renewing itself. Cells divide and grow in the deepest layer called the "stratum germinativum", and the newly formed cells are displaced outwards as the newly forming cells are grown beneath them. As the newly formed cells pass through the "stratum granulosum" they begin to die and lose their nucleus material. During their outward journey the newly formed cells degenerate further into layers of the flat keratinous material, which forms the "stratum corneum". This layer consists of dead cells, and the epidermis layer, therefore, is a constantly changing layer of skin. As such when the "stratum corneum" it removed it will regenerate within twenty four hours. The deeper layers of skin consist of the vascular and nervous components of the skin, sweat glands, sweat ducts and hair follicles.

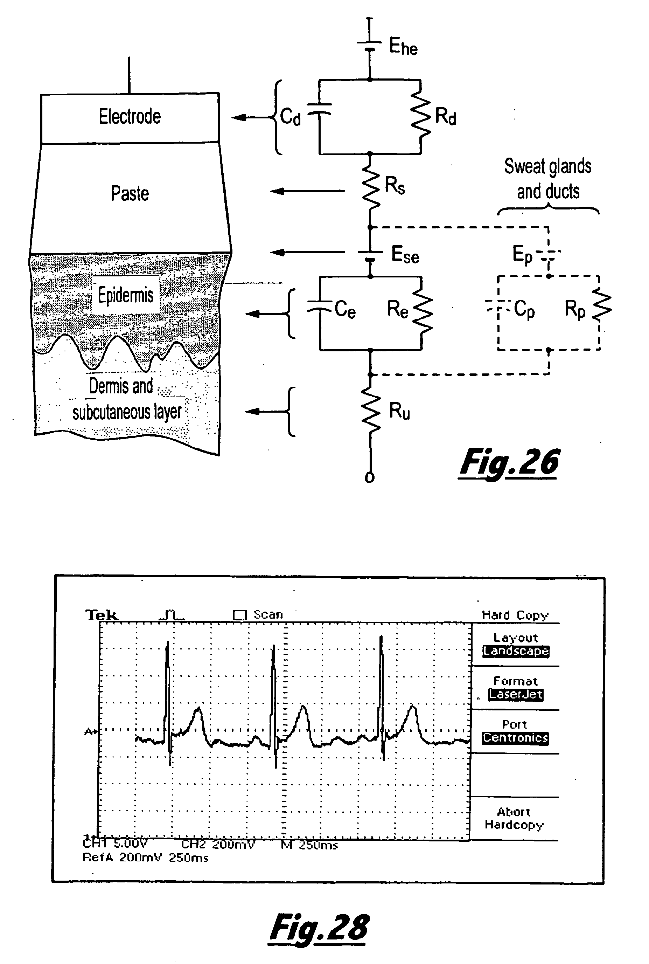

An equivalent electrical circuit of the skin-electrode interface is shown in Figure 26, in which:

$E_{hc}$ is the half cell potential between the electrode and electrolyte interface;
$C_d$ is the capacitance of the electrode electrolyte interface;
$R_d$ is the leakage resistance of the electrode electrolyte interface;
$R_s$ is the electrolyte resistance;
$E_{se}$ is the half cell potential between the electrolyte and the skin;
$C_e$ is the capacitance of the skin electrolyte interface;
$R_e$ is the leakage resistance of the electrolyte skin interface;
$R_u$ is the resistance of the dermis and subcutaneous layer;
$E_p$ is the half cell potential of sweat glands, ducts and the electrolyte;
$C_p$ is the capacitance of sweat glands, ducts and the electrolyte interface; and
$R_p$ is the leakage resistance of sweat glands, ducts and the electrolyte interface.

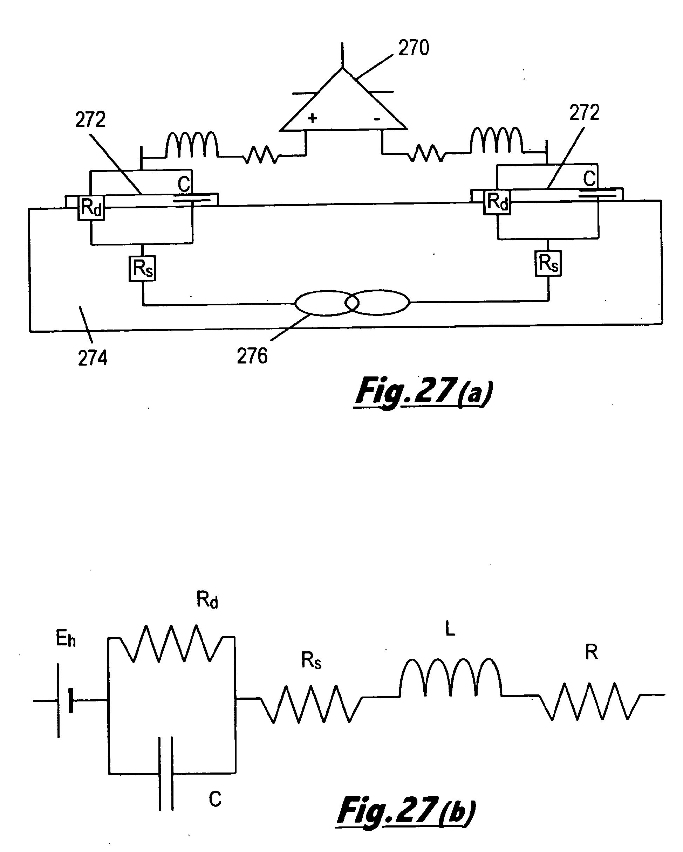

The electrical equivalent circuit of the ECA system was developed by modifying the known Cole-Cole model with an inductance (L) and a resistance (R) connected in series; this represents the impedance of the electrical pathways connecting the ECA to a pre-processing unit. The lump components $F_d$, $R_s$, L, R and $C_d$ were determined by analysing the impedance spectrum of the ECA system. The impedance is measured between two electrodes. The electrode area is selected to be 2.5 x 2.5 $cm^2$ (or course, this area is in no way limiting). The equivalent circuit of the measuring arrangement shown in the Figure 27(a), and comprises a pre-amplifier 270 and, knitted electrodes 272. The skin tissue and an effective ECG source are shown at 274 and 276, respectively.

The total impedance of the electrode system can be represented by the equivalent circuit shown in Figure 27 (b). The individual components were estimated empirically as demonstrated by Danchev and A1 Hatib. (Danchev, S., and A1 Hatib, F., "Non linear curve fitting for bio electrical impedance data analysis: a minimum ellipsoid volume method" Physiological measurement 20 (1999) N1-N9, the contents of which are incorporated herein by reference).

## Determination of electrode-skin-electrode impedance

A sleeve integrated with electrodes was produced for impedance measurements. In this example the seamless knitted sleeve the electrodes (contact area 2.5 x 2.5$cm^{2)}$ positioned 5cm apart. No skin penetration was carried out. The electrodes were connected to an impedance analyzer, and the impedance was measured within the frequency spectrum of the ECG, ie, between 5 - 400 Hz.

Current clinical practice for measuring an ECG is to attach metal electrodes to the skin of a patient. Prior to attaching the electrodes a thin layer of electro conductive gel is applied to the surfaces of the electrodes, in order to improve the electrical conductivity between the skin - electrode interface. The performance of ECAs of the invention was studied using this practice by employing a thin layer of electro conductive gel. Impedance measurements were made which demonstrated a value between 7 - 9 KOhms for the amplitude and a phase angle variation between -35° and -65°.

An example of an ECG signal picked up by the ECA system is shown in Figure 28. The signal was of sufficient quality for early diagnostic purposes. The PQRST components could be clearly identified in the wave forms; occasionally the U wave could also be observed.

Measurements were also performed without the electro conductive gel. The magnitude of the impedance was in the range 1.4 MOhms to 200KOhms for the frequency range 20 - 40Hz. The phase angle was in the range of -55° to -75°. ECG signals could be recovered using this system. A certain amount of noise is observed in the "raw" ECG signal due to artefacts such as deformation of the knitted structure and line interference. Signal processing techniques can be used advantageously to remove such artefacts and interferences, and thus improve the quality of the ECG signal.

**Garments incorporating knitted transducer devices**

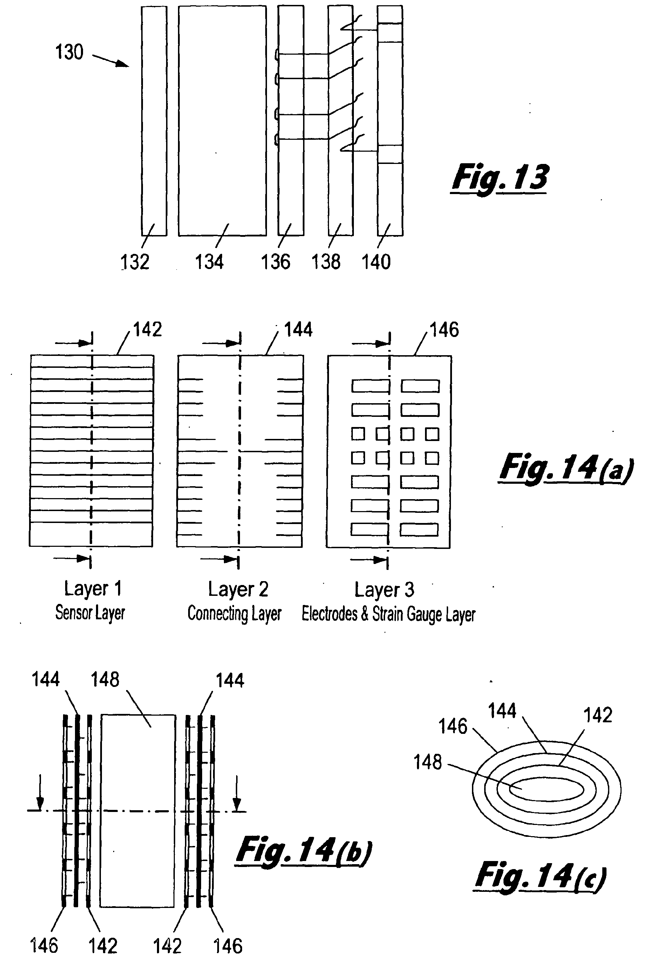

A wide range of garments may be produced which incorporate knitted transducer devices of the type described above. Examples include gloves, mitts, socks, trousers, and pants. Particularly useful examples include garments for the upper body, such as vests, sweaters, shirts and the like. Figure 13 shows a cross sectional view of a vest 130 which incorporates knitted transducer devices. The back portion of the vest is comprised of a single knitted layer 132. In contrast, the front portion of the vest is made up of three separate knitted layers, namely a first layer 136, a second layer 138, and a third layer 140. Also shown in Figure 13 in somewhat schematic fashion is the body 134 of a wearer of the vest. We describe the vest shown in Figure 13 as a (1 + 3) layer garment. Different transducers may be knitted into different layers. In a preferred, but non-limiting embodiment, transducers suitable for ECG measurement are knitted into the first layer 136, ie, the layer next to the skin of the wearer; further transducers such as strain gauges can be knitted into the third layer 140. It is noted that it can be desirable to incorporate inductive knitted transducers into the garment. Such transducers are powered at high frequencies such as 1 MHZ or more in order to improve the signal to noise ratio. Such high frequency signals can be harmful to the human body and thus the configuration shown in Figure 13, in which a second layer 138 is disposed between the third layer 140 and the wearer 134 is advantageous. The second layer 138 acts to screen the wearer from the high frequency signals. Additionally, or alternatively, the second layer 138 can provide a further useful function by providing connecting leads which are in connection with transducers in the first and/or third layers 136, 140. These connecting leads consist of metal yarns which form part of the knit of the second layer 138. In particular, the knitted rows (courses) of the second layer 138 can comprise metal yarns. It is noted that biopotential electrodes for ECG measurements are required to be in contact with the skin, but their connecting leads should not. Thus, the configuration shown in Figure 13 is particularly advantageous when the transducers in the first layer 136 are biopotential electrodes. The transducers are connected, via the connecting leads, to suitable power supply/detector means. Such means are well known in the art and thus are not further exemplified herein.

Figure 14 depicts an embodiment related to the embodiment of Figure 13. The embodiment of Figure 14 differs in that both front and back portions of the vest are made up of three knitted layers. Thus, using our nomenclature, Figure 14 shows a (3 + 3) layer vest. Both front and back portions of the vest comprise a first knitted layer 142 having a plurality of transducers disposed therein as part of its knitted structure, a second knitted layer 144 having conductive yarns which act as connectors for the transducers located in the vest, and a third kitted layer 146 having further transducers disposed therein as part of its knitted structure. In a preferred, but non-limiting embodiment, the transducers in the first knitted layer 142 are electrodes and the transducers in the third layer 146 are strain gauges. Also shown in Figure 14, again in somewhat schematic form, is the body 148 of a wearer of the vest. The majority of the vests can be knitted using conventional non- conductive yarn. Lycra is an example of a suitable yarn, although many other candidates would suggest themselves to the skilled person.

**Fashioned vest having a total of three layers (1 + 2 layers) with no seams and knitted in sensors.**

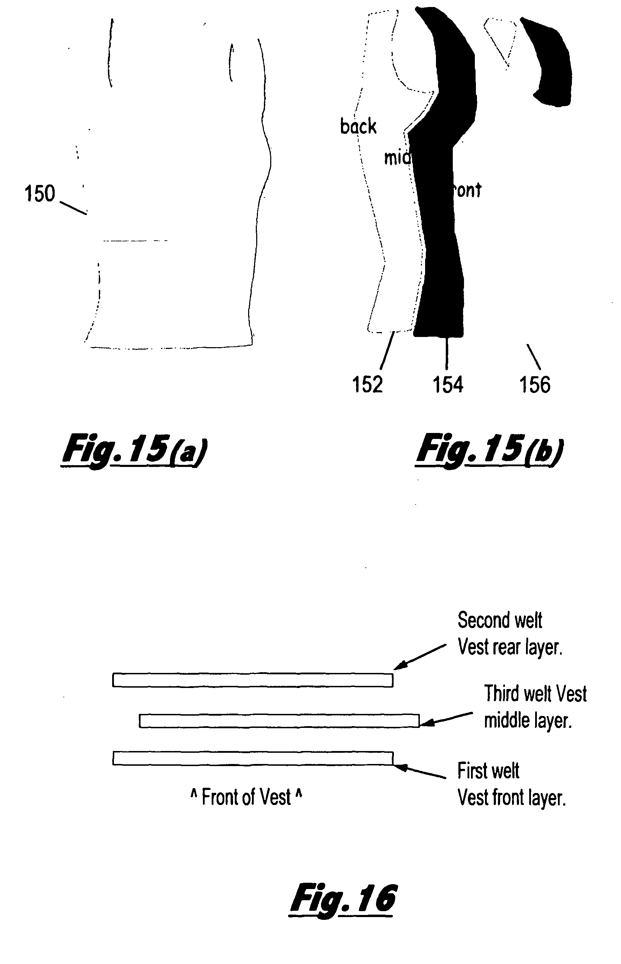

Figure 15 shows a three layer fashioned vest garment 150 with a back 152, middle 154 and front 156 layer, no seams and having knitted in sensors (transducers), for example for use in taking ECG heart readings and respiratory readings. This garment can be used within a hospital environment, also in the world of sport to collect data from the patient/person wearing the garment. The garment can also be washed, donned and worn by unskilled operatives as the sensors have been knitted into the garment in their correct positions, and thus require no special alignment or treatment.

Three separate welts, one for each layer of fabric, are used so that there are no raw edges on the completed vest, eliminating over locking these three edges of the fabric.

The following is the knitting sequence for the three layered welt:

A draw thread divides the comb waste from the three welts. Each layer of the vest garment starts with its own welt, so that there are no raw edges.

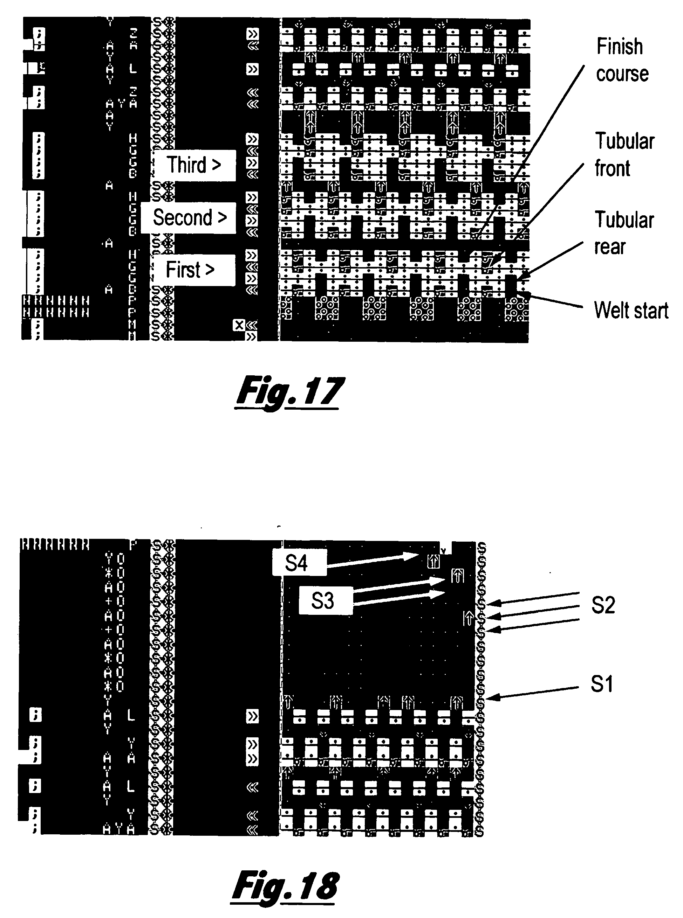

Each welt consists of one knitted course of one in four stitches on the front needle bed and also one in four stitches on the rear needle bed (Figure 17 welt start). The next knitted course knits exactly the same layout of needles but only on the rear needle bed (Figure 17 tubular rear), and the next knitted course knits exactly the same layout of needles but only on the front needle bed (Figure 17 tubular front). These two courses can be repeated if necessary. The operation is then finished with knitting the same needle layout as the welt start course. The needle loops on the rear bed are now transferred to the front needles, so that they are out of the way and allow the second welt to begin.

This second welt is knitted in the same sequence as the first welt, except that the loop layout starts one needle to the left, the last knitted front loops are transferred to the rear needles; this allows the third welt to be knitted inside the two outer layers of fabric (Figure 16).

The third welt again is knitted in the same sequence as the first and second welt; it also starts one needle to the left with the rear loops but the front loops knit on the empty needles opposite the second welt rear loops. After the welt sequence is finished the front loops of the last knitted course are transferred to the rear.

To achieve a fashioned three layer vest garment the front, middle and back panels of fabric should be connected, except for the start of the arms and neck, where the back panel still has to be separate up to the bind off at the shoulder.

The following is the knitting and transference sequence for the fashioning of all three layers on the right selvedge of the vest and for the joining of the front and middle panels. Reference is made to Figure 18.

After the completion of the main body knitting sequence:

Stage 1: the right hand rear loop (middle layer of the vest) is transferred two needles to the left from the selvedge, in order to allow the needle to be able to receive the loop from the front layer of the vest;

Stage 2: the right hand front loop (front layer of the vest) is transferred in the ground position directly to the rear. This same loop is then transferred to the left from the selvedge by two needles; doubling up the second front loop of the front layer, this completes the front layer fashioning;

Stage 3: the right hand rear loop (rear layer of the vest) is transferred in the ground position directly to the now empty needle at the front. This same loop is then transferred to the left from the selvedge by two needles doubling up the second needle loop in from the selvedge. This completes the rear layer fashioning;

Stage 4: the needle that was moved at the beginning (Stage 1) to allow other loops to be transferred is now transferred to the left on top of the original right selvedge needle loop. This completes the middle layer fashioning.

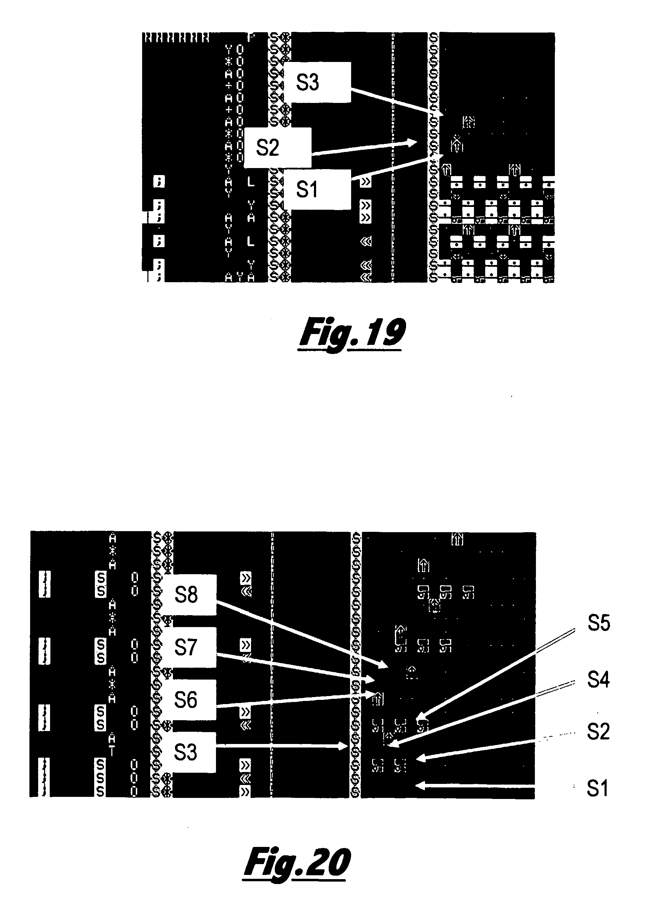

The following is the knitting and transference sequence for the fashioning of all three layers on the left selvedge of the vest, and also for the joining of the front and middle panels. Reference is made to Figure 19.

After completion of the knitting sequence:

Stage 1: the first loop on the left selvedge of the middle layer of the vest is transferred to an empty needle on the front by two needles to the right - this is to allow the needle to be able to receive the loop from the front layer.

Stage 2: the first loop from the left selvedge of the front layer is transferred to the rear in the ground position. This is then transferred with the first loop of the rear layer two needles to the right. This completes the front layer fashioning and half of the rear layer fashioning.

Stage 3: the first loop to be transferred at Stage 1 of the middle layer is now transferred to the rear, doubling the second needle loop from the left selvedge.

The bind off and finish of the straps is towards the back of the shoulder. This is so that the double layer of fabric lies across the shoulder, and helps with the comfort of the vest when it is being worn. Reference is made to Figure 20.

The following is the knitting and transference sequence for the bind off and finish of the straps at the shoulder:

Stage 1: the main yam knits three odd needles on the rear needle bed from the left, and then knits the same three needles out towards the left (Figure 20).

Stage 2: the main yam knits two even needles in from the left and leaves the yarn feeder on the right of the binding off area (Figure 20).

Stage 3: the first needle on the left is now transferred one needle to the right, this doubles up the left needle loop on the front needle bed (figure 20).

Stage 4: from the odd needles on the front needle bed, one loop on the left of the middle layer is now transferred in the ground position to the rear (Figure 20).

Stage 5: the main yarn knits three even needles on the front from the right, and then knits three odd needles on the rear towards the right, leaving the yam feeder on the right of the binding off area (Figure 20).

Stage 6: the left loop of the front layer is transferred to an empty needle on the rear in ground position (Figure 20).

Stage 7: the previous left front layer loop transferred at Stage 6 and the left rear loops are transferred to two needles to the right on to the front needles (Figure 20).

Stage 8: the second loop in from the left on the front needle bed is transferred in ground position to the rear, doubling up the left hand loop of the rear layer (Figure 20).

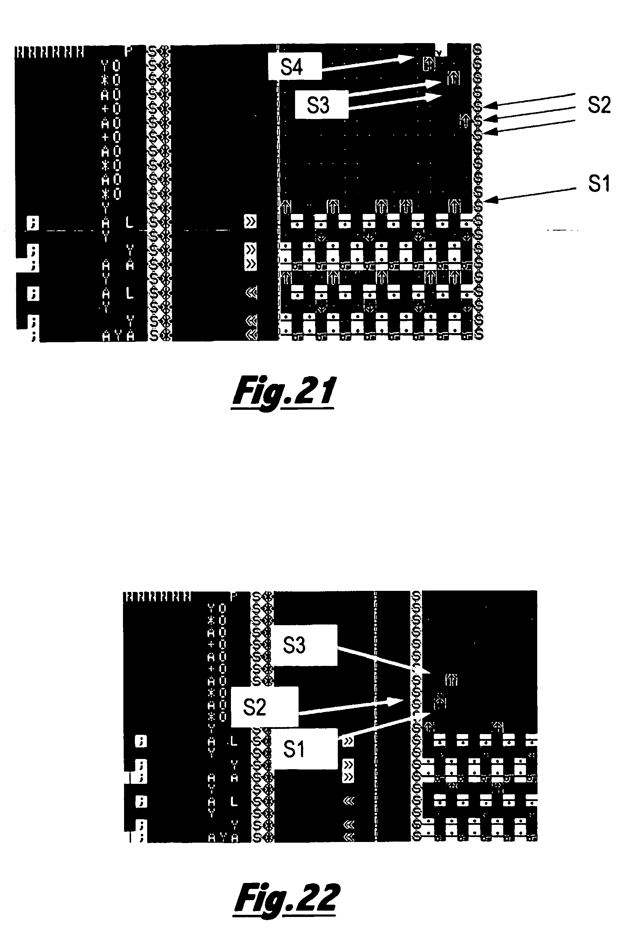

The following is the knitting and transference sequence for the fashioning of all three layers for the left middle neck opening of the vest, and also for the joining of the front and middle panels. Reference is made to Figure 21.

After completion of the main body knitting sequence:

Stage 1: the right hand middle rear loop (middle layer of the vest) is transferred two needles to the left from the left middle selvedge, this is to allow the needle to be able to receive the loop from the front layer of the vest (Figure 21).

Stage 2: the right hand middle front loop (front layer of the vest) is transferred in the ground position directly to the rear. This same loop is then transferred to the left from the right middle selvedge by two needles; doubling up the second front loop of the front layer, this completes the front layer fashioning (Figure 21).

Stage 3: the right hand middle rear loop (rear layer of the vest) is transferred in the ground position directly to the now empty needle at the front. This same loop is then transferred to the left from the right middle selvedge by two needles doubling up the second needle loop in from the selvedge. This completes the rear layer fashioning (Figure 21).

Stage 4: the needle that was moved at the beginning (Stage 1) to allow other loops to be transferred is now transferred to the left on top of the original right middle selvedge needle loop. This completes the middle layer fashioning (Figure 21).

The following is the knitting and transference sequence for the fashioning of all three layers for the right middle neck opening of the vest, also for joining of the front and middle panels. Reference is made to Figure 22.

After completion of the knitting sequence:

Stage 1: the first loop on the right middle selvedge of the middle layer of the vest is transferred to an empty needle on the front by two needles to the right, this is to allow the needle to be able to receive the loop from the front layer (Figure 22).

Stage 2: the first loop from the right middle selvedge of the front layer is transferred to the rear in the ground position. This is then transferred with the first loop of the rear layer two needles to the right. This completes the front layer fashioning and half of the rear layer fashioning (Figure 22).

Stage 3: the first loop to be transferred at stage 1 of the middle layer is now transferred to the rear, doubling the second needle loop from the selvedge (Figure 22).

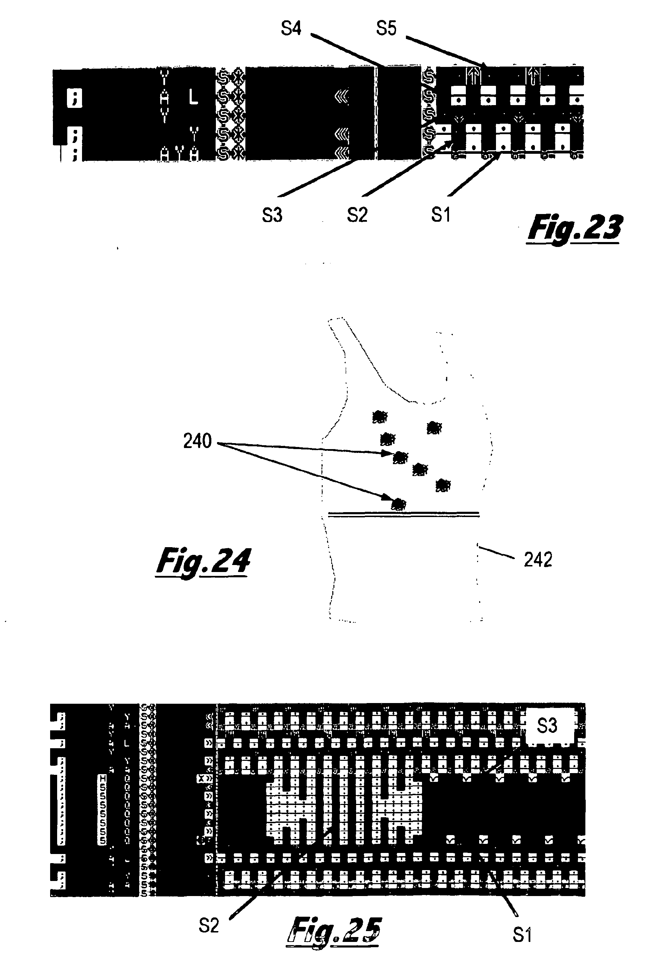

The following is the knitting and transference sequence for the completion of the knitting sequence for the main body of the three layer vest. Reference is made to Figure 23.

Stage 1: the first feeder knits even needles on the front bed only (front layer of fabric).

Stage 2: the second feeder knits even needles on the rear bed only (middle layer of fabric).

Stage 3: the middle layer loops are now transferred to the front needle bed one needle to the left - this is to allow the rear layer to be knitted.

Stage 4: the third yarn feeder knits odd needles on the rear (rear layer of fabric).

Stage 5: the middle layer loops are now transferred to the rear needle bed one needle to the right, this is to allow the middle and front layers to be knitted.

The Figure 24 shows an example layout of sensors 240 knitted into the middle layer 242 of the garment, which sensors can be knitted in any position required.

The following is the knitting sequence for an example garment only. Reference is made to Figure 25. It is an example only, since the sensor can be knitted in different shapes, such as circular, oblong, or square according to the type of yarn and signals required from them. Different types of sensor can be employed depending on the end application. The middle layer of the vest is used to house the sensor because it is next to the skin, the front layer of the vest being used to bring in and out the sensor yarn so that it is insulated by the middle layer. The sensor connector leads are knitted with a highly conductive (low specific resistance) yarn and these knitted rows (courses) form the conductive pathways of an integrated circuit.

Stage 1: the main three layers of the vest are now in their normal position on the rear and middle layers on the rear needle bed, and the front layer on the front needle bed. At this point the feeder with the sensor yarn is knitted or tucked (in this case) from the right to the start of the sensor position on the front layer front needles and knits only the starting width of the sensor on the middle layer rear needles (Figure 25).

Stage 2: at this point the feeder with the sensor yarn is knitted to the right and then to the left following the selection required to knit the shape or type of sensor required (Figure 25).

Stage 3: when the sensor has been completely knitted, then the yarn feeder with the sensor yarn knits to the right of the sensor and is then knitted or tucked (in this case) to the right selvedge (Figure 25).

Non-limiting advantages and features of the present invention are as follows:

•    the creation of a seamless multilayer garment using electronic flat-bed knitting technology, which enables garments to be knitted with a range of different transducers, for example electrodes, strain gauges, thermocouples for temperature measurement, proximity sensors (capacitive sensors), knitted microphones and antennae. The above transducers and electrodes can be knitted into different layers of the garment;

- arrays of transducers and electrodes can be knitted with the garment, which would enable the transducers and electrodes to be selected for their optimum performance using intelligent software;
- the transducers, electrodes and their connecting leads (conductive paths) can be knitted as one integral knitted structure which could be a single or a multilayer (two or more layers). In the case of electrodes the sensing patch (the electrode) can be knitted on to one layer and the connecting leads (conductive path) knitted on to the next layer. This may be achieved with electronic flat-bed knitting technology;
- intarsia and jacquard techniques may be used to create the transducers, electrodes and conductive paths. This enables the knitting of electrodes and transducers of different geometrical shapes and sizes (rectangular, circular, elliptical etc.). Electronic flat-bed technology also allows knitting of an array of transducers and electrodes of different geometrical shapes and sizes, which enable us to measure different physiological values;
- the concept of knitting a garment such as a multilayer vest enables the creation of a health monitoring system as an integrated circuit. The multilayer technique also enables us to incorporate microelectronic circuits and/or components between layers;
- garments can be constructed with connectors, transducers, electrodes, antennae and electrical shielding as one structure (a complex 3D seamless knitted structure);
- the electrodes and transducers could be knitted on to the front and back layers of the garment. The different transducers and electrodes are located at positions that allow the best quality signals to be captured.

There are numerous variations to the embodiments discussed above which are within the scope of the invention. For example, a garment may incorporate further knitted layers.

### Claims

1. A knitted structure including as a part thereof a transducer device having at least one transduction zone knitted with electrically conductive fibres so that deformation of the device results in a variation of an electrical property of the transduction zone, and means for monitoring such variations to provide an indication of deformations of the knitted structure, **characterised in that** in said structure the first and last courses of the transduction zone are knitted with electrically conductive fibres which act as conducting leads for the transducer device.

2. A knitted structure according to Claim 1 in which the transduction zone is knitted with combinations of binding elements selected from the group comprising stitches, tuck loops and floats.

3. A knitted structure according to Claim 1 or Claim 2 in which the electrically conductive fibres comprise elastomeric conductive yarn.

4. A knitted structure according to any preceding Claim in which the transduction zone is knitted with a plurality of types of electrically conductive fibres, each type having a different resistivity.

5. A knitted structure according to any preceding Claim in which deformation of the transducer device results in a variation of the electrical resistance of the transduction zone.

6. A knitted structure according to Claim 5 operable as a strain gauge, in which the transduction zone is knitted with electrically conductive fibres and non-conductive fibres; and the electrically conductive fibres in the transduction zone extend in a common direction.

7. A knitted structure according to Claim 6 in which the electrically conductive fibres extend in the course direction of the transduction zone.

8. A knitted structure according to Claim 6 or Claim 7 in which the electrically conductive fibres are incorporated into the transduction zone as laid in fibres.

9. A knitted structure according to Claim 5 in which displacement of the transducer from a relaxed configuration results in a variation of the electrical resistance of the transduction zone which is functionally related to the displacement.

10. A knitted structure according to Claim 9 when dependent on Claim 4 in which the transduction zone comprises:

a transducing section formed from knitting together electrically conductive fibres; and

a plurality of electrical contacts in electrical connection with the transducing section, the electrical contacts comprising knitted electrically conductive fibres of a higher electrical conductivity than the electrical conductivity of the electrically conductive fibres in the transducing zone.

11. A knitted structured according to any one of Claims 1 to 4 in which deformation or movement of the transducer device results in a variation of the inductance of the transduction zone.

12. A substantially cylindrical inductive solenoid knitted structure according to Claim 11.

13. A knitted structure according to Claim 12 in which the transduction zone is knitted from electrically conductive fibres and non-conductive fibres.

14. A knitted structure according to Claim 13 in which the electrically conductive fibres are disposed on the outside of the solenoid and the non-conductive fibres are disposed on the inside of the solenoid.

15. A knitted structure according to any of Claims 1 to 4 in which deformation of the transducer device results in a variation of the electrical capacitance of the transduction zone.

16. A knitted structure according to Claim 15 in which the electrically conductive fibres in the transduction zone define a plurality of spaced apart electrodes.

17. A knitted structure according to Claim 16 in which the electrodes are concentric.

18. A knitted structure according to Claim 16 in which the electrodes are interdigitated.

19. A knitted structure according to Claim 16 in which the electrodes are parallel plate electrodes.

20. A knitted structure according to Claim 19 in which the transduction zone includes one or more knitted layers of non-conductive fibres extending between the parallel plate electrodes.

21. A knitted structure according to any preceding Claim wherein the transducer device comprises a plurality of knitted layers.

22. A knitted structure according to any of Claims 1 to 21 including a plurality of said transducer devices.

23. A detection system comprising a knitted structure according to any preceding Claim;
electrical supply means for supplying an electrical signal to the knitted transducer device; and
detection means for monitoring an electrical characteristic of said at least one knitted transducer device.

24. A system according to Claim 23 including a plurality of the knitted transducer devices, and in which the detection means is adapted to derive information pertaining to the relative spatial orientation of the transducer devices.

25. A system according to Claim 24 in which the detection means derives information pertaining to the relative orientations of the knitted transducer devices by comparing monitored electrical characteristics of the knitted transducer devices.

26. A system according to any of Claims 23 to 25 in which electrical characteristics are monitored at a plurality of frequencies.

27. A system according to Claim 26 in which the electrical characteristics are monitored at a plurality of frequencies in the range 1 to 1000Hz.

28. A system according to Claim 26 or Claim 27 in which the detection means produces an ECG from the monitored electrical characteristics.

29. A garment comprising a knitted structure according to any of Claims 1 to 22.

30. A garment according to Claim 29 comprising a plurality of knitted layers.

31. A garment according to Claim 30 in which the knitted layers are formed as an integral knitted structure.

32. A garment according to Claim 30 or Claim 31 comprising a first knitted layer having at least one knitted transducer device and in which the second knitted layer is knitted with electrically conductive fibres which act as connecting leads for the knitted transducer device.

33. A garment according to any of Claims 30 to 32 comprising three or more knitted layers.

34. A garment according to Claim 33 comprising a first knitted layer having one or more knitted transducer devices; a second knitted layer; and a third knitted layer having one or more knitted transducer devices.

35. A garment according to Claim 34 in which the second knitted layer is adapted to screen electrical signals emanating from or introduced to a knitted transducer device located in the third knitted layer.

36. A garment according to Claim 35 in which a knitted transducer device in the third layer is an inductive knitted transducer device.

37. A seamless garment according to any of Claims 29 to 36.

**Patentansprüche**

1. Gestrickte Struktur, die als Teil von ihr aufweist: eine Wandlervorrichtung mit mindestens einer Wandlungszone, die mit elektrisch leitfähigen Fasern gestrickt ist, so daß eine Verformung der Vorrichtung zu einer Änderung einer elektrischen Eigenschaft der Wandlungszone führt, und Mittel zum Überwachen solcher Änderungen, um eine Angabe über Verformungen der gestrickten Struktur bereitzustellen, **dadurch gekennzeichnet, daß** in der Struktur die erste und letzte Maschenreihe der Wandlungszone mit elektrisch leitfähigen Fasern gestrickt ist, die als leitende Zuleitungen für die Wandlervorrichtung wirken.

2. Gestrickte Struktur nach Anspruch 1, in der die Wandlungszone mit Kombinationen aus Bindeelementen gestrickt ist, die aus der Gruppe gewählt sind, die Maschen, Fanghenkeln und Flottungen umfaßt.

3. Gestrickte Struktur nach Anspruch 1 oder 2, in der die elektrisch leitfähigen Fasern leitfähiges Elastomergarn umfassen.

4. Gestrickte Struktur nach einem der vorhergehenden Ansprüche, in der die Wandlungszone mit einer Vielzahl von Typen von elektrisch leitfähigen Fasern gestrickt ist, wobei jeder Typ einen anderen spezifischen Widerstand hat.

5. Gestrickte Struktur nach einem der vorhergehenden Ansprüche, in der eine Verformung der Wandlervorrichtung zu einer Änderung des elektrischen Widerstands der Wandlungszone führt.

6. Gestrickte Struktur nach Anspruch 5, die als Dehnungsmesser betriebsfähig ist und in der die Wandlungszone mit elektrisch leitfähigen Fasern und nichtleitfähigen Fasern gestrickt ist; und die elektrisch leitfähigen Fasern in der Wandlungszone sich in einer gemeinsamen Richtung erstrecken.

7. Gestrickte Struktur nach Anspruch 6, in der die elektrisch leitfähigen Fasern sich in der Maschenreihenrichtung der Wandlungszone erstrecken.

8. Gestrickte Struktur nach Anspruch 6 oder 7, in der die elektrisch leitfähigen Fasern als Einlegefasern in die Wandlungszone eingearbeitet sind.

9. Struktur nach Anspruch 5, in der eine Verschiebung des Wandlers aus der entspannten Konfiguration zu einer Änderung des elektrischen Widerstands der Wandlungszone führt, die mit der Verschiebung in funktionaler Beziehung steht.

10. Gestrickte Struktur nach Anspruch 9, sofern abhängig von Anspruch 4, in der die Wandlungszone umfaßt:

ein Wandlungsteilstück, das durch gemeinsames Verstricken von elektrisch leitfähigen Fasern ausgebildet ist;

und

eine Vielzahl von elektrischen Kontakten in elektrischer Verbindung mit dem Wandlungsteilstück, wobei die elektrischen Kontakte gestrickte elektrisch leitfähige Fasern einer höheren spezifischen elektrischen Leitfähigkeit umfassen als die spezifische elektrische Leitfähigkeit der elektrisch leitfähigen Fasern in der Wandlungszone.

11. Gestrickte Struktur nach einem der Ansprüche 1 bis 4, in der eine Verformung oder Bewegung der Wandlervorrichtung zu einer Änderung der Induktivität der Wandlungszone führt.

12. Im wesentlichen zylindrische leitfähige gestrickte Spulenstruktur nach Anspruch 11.

13. Gestrickte Struktur nach Anspruch 12, in der die Wandlungszone aus elektrisch leitfähigen Fasern und nichtleitfähigen Fasern gestrickt ist.

14. Gestrickte Struktur nach Anspruch 13, in der die elektrisch leitfähigen Fasern sich auf der Außenseite der Spule befinden und die nichtleitfähigen Fasern sich auf der Innenseite der Spule befinden.

15. Gestrickte Struktur nach einem der Ansprüche 1 bis 4, in der eine Verformung der Wandlervorrichtung zu einer Änderung der elektrischen Kapazität der Wandlungszone führt.

16. Gestrickte Struktur nach Anspruch 15, in der die elektrisch leitfähigen Fasern in der Wandlungszone eine Vielzahl von voneinander beabstandeten Elektroden definierten.

17. Gestrickte Struktur nach Anspruch 16, in der die Elektroden konzentrisch sind.

18. Gestrickte Struktur nach Anspruch 16, in der die Elektroden verschränkt sind.

19. Gestrickte Struktur nach Anspruch 16, in der die Elektroden Parallelplattenelektroden sind.

20. Gestrickte Struktur nach Anspruch 19, in der die Wandlungszone eine oder mehrere gestrickte Schichten aus nichtleitfähigen Fasern aufweist, die sich zwischen den Parallelplattenelektroden erstrecken.

21. Gestrickte Struktur nach einem der vorhergehenden Ansprüche, wobei die Wandlervorrichtung eine Vielzahl von gestrickten Schichten umfaßt.

22. Gestrickte Struktur nach einem der Ansprüche 1 bis 21, mit einer Vielzahl der Wandlervorrichtungen.

23. Detektionssystem, umfassend: eine gestrickte Struktur nach einem der vorhergehenden Ansprüche; elektrisches Zuführungsmittel zur Zuführung eines elektrischen Signals zu der gestrickten Wandlervorrichtung; und Detektionsmittel zur Überwachung einer elektrischen Charakteristik der zumindest einen gestrickten Wandlervorrichtung.

24. System nach Anspruch 23, das eine Vielzahl der gestrickten Wandlervorrichtungen aufweist und in dem das Detektionsmittel dafür eingerichtet ist, Information bezüglich der relativen räumlichen Orientierung der Wandlervorrichtungen abzuleiten.

25. System nach Anspruch 24, in dem das Detektionsmittel Information bezüglich der relativen Orientierungen der gestrickten Wandlervorrichtungen durch Vergleichen von überwachten elektrischen Charakteristiken der gestrickten Wandlervorrichtungen ableitet.

26. System nach einem der Ansprüche 23 bis 25, in dem elektrische Charakteristiken mit einer Vielzahl von Frequenzen überwacht werden.

27. System nach Anspruch 26, in dem die elektrischen Charakteristiken mit einer Vielzahl von Frequenzen im Bereich von 1 bis 1000 Hz überwacht werden.

28. System nach Anspruch 26 oder 27, in dem das Detektionsmittel ein EKG aus den überwachten elektrischen Charakteristiken erzeugt.

**29.** Kleidungsstück mit einer gestrickten Struktur nach einem der Ansprüche 1 bis 22.

**30.** Kleidungsstück nach Anspruch 29, das eine Vielzahl von gestrickten Schichten umfaßt.

**31.** Kleidungsstück nach Anspruch 30, in dem die gestrickten Schichten als einstückige gestrickte Struktur ausgebildet sind.

**32.** Kleidungsstück nach Anspruch 30 oder 31, das eine erste gestrickte Schicht mit mindestens einer gestrickten Wandlervorrichtung umfaßt und in dem die zweite gestrickte Schicht mit elektrisch leitfähigen Fasern gestrickt ist, die als Verbindungsleitungen für die gestrickte Wandlervorrichtung wirken.

**33.** Kleidungsstück nach einem der Ansprüche 30 bis 32, das drei oder mehr gestrickte Schichten umfaßt.

**34.** Kleidungsstück nach Anspruch 33, umfassend: eine erste gestrickte Schicht mit einer oder mehreren gestrickten Wandlervorrichtungen; eine zweite gestrickte Schicht; und eine dritte gestrickte Schicht mit einer oder mehreren gestrickten Wandlervorrichtungen.

**35.** Kleidungsstück nach Anspruch 34, in dem die zweite gestrickte Schicht dafür angepaßt ist, elektrische Signale auszuwählen, die von einer in der dritten gestrickten Schicht befindlichen gestrickten Wandlervorrichtung ausgehen oder in diese hineingeleitet werden.

**36.** Kleidungsstück nach Anspruch 35, in dem eine gestrickte Wandlervorrichtung in der dritten Schicht eine induktive gestrickte Wandlervorrichtung ist.

**37.** Nahtloses Kleidungsstück nach einem der Ansprüche 29 bis 36.

## Revendications

**1.** Structure tricotée incluant, en tant que partie de celle-ci un dispositif de transducteur qui comporte au moins une zone de transduction tricotée avec des fibres électriquement conductrices de telle sorte qu'une déformation du dispositif aboutisse à une variation d'une propriété électrique de la zone de transduction, et un moyen pour surveiller ces variations afin de fournir une indication de déformation de la structure tricotée, **caractérisée en ce que,** dans ladite structure, les premier et dernier passages de la zone de transduction sont tricotés avec des fibres électriques non conductrices qui jouent le rôle de connexions de conduction pour le dispositif transducteur.

**2.** Structure tricotée selon la revendication 1, dans laquelle la zone de transduction est tricotée avec des combinaisons d'éléments de liaison choisis parmi le groupe comprenant des points de liage, des boucles de charge et des flottés.

**3.** Structure tricotée selon la revendication 1 ou 2, dans laquelle les fibres électriquement conductrices comprennent un fil conducteur élastomérique.

**4.** Structure tricotée selon l'une quelconque des revendications précédentes, dans laquelle la zone de transduction est tricotée avec une pluralité de types de fibres électriquement conductrices, chaque type présentant une résistivité différente.

**5.** Structure tricotée selon l'une quelconque des revendications précédentes, dans laquelle la déformation du dispositif de transducteur aboutit une variation de la résistance électrique de la zone de transduction.

**6.** Structure tricotée selon la revendication 5, pouvant fonctionner en tant que jauge de contrainte, dans laquelle la zone de transduction est tricotée avec des fibres électriquement conductrices et des fibres non conductrices, et les fibres électriquement conductrices dans la zone de transduction s'étendent dans une direction commune.

**7.** Structure tricotée selon la revendication 6, dans laquelle les fibres électriquement conductrices s'étendent dans la direction de passe de la zone de transduction.

**8.** Structure tricotée selon la revendication 6 ou 7, dans laquelle les fibres électriquement conductrices sont incorporées dans la zone de transduction en tant que fibres d'insertion.

**9.** Structure tricotée selon la revendication 5, dans laquelle un mouvement du transducteur par rapport à une configuration relaxée aboutit à une variation de la résistance électrique de la zone de transduction, laquelle est rapportée fonctionnellement au mouvement.

**10.** Structure tricotée selon la revendication 9 lorsqu'elle dépend de la revendication 4, dans laquelle la zone de transduction comprend:

une section de transduction formée en tricotant ensemble des fibres électriquement conductrices; et
une pluralité de contacts électriques en connexion électrique avec la section de transduction, les contacts électriques comprenant des fibres électriquement conductrices tricotées d'une conductivité électrique plus élevée que la conductivité électrique des fibres électriquement conductrices dans la zone de transduction.

**11.** Structure tricotée selon l'une quelconque des revendications 1 à 4, dans laquelle une déformation ou un mouvement du dispositif transducteur aboutit à une variation de l'inductance de la zone de transduction.

**12.** Structure tricotée d'électro-aimant inductif sensiblement cylindrique selon la revendication 11.

**13.** Structure tricotée selon la revendication 12, dans laquelle la zone de transduction est tricotée à partir de fibres électriquement conductrices et de fibres non conductrices.

**14.** Structure tricotée selon la revendication 13, dans laquelle les fibres électriquement conductrices sont disposées sur l'extérieur de l'électro-aimant et les fibres non conductrices sont disposées sur l'intérieur de l'électro-aimant.

**15.** Structure tricotée selon l'une quelconque des revendications 1 à 4, dans laquelle la déformation du dispositif de transducteur aboutit à une variation de la capacité électrique de la zone de transduction.

**16.** Structure tricotée selon la revendication 15, dans laquelle les fibres électriquement conductrices dans la zone de transduction définissent une pluralité d'électrodes espacées.

**17.** Structure tricotée selon la revendication 16, dans laquelle les électrodes sont concentriques.

**18.** Structure tricotée selon la revendication 16, dans laquelle les électrodes sont interdigitées.

**19.** Structure tricotée selon la revendication 16, dans laquelle les électrodes sont des électrodes en plaques parallèles.

**20.** Structure tricotée selon la revendication 19, dans laquelle la zone de transduction inclut une ou plusieurs couches tricotées de fibres non conductrices s'étendant entre les électrodes en plaques parallèles.

**21.** Structure tricotée selon l'une quelconque des revendications précédentes, dans laquelle le dispositif de transducteur comprend une pluralité de couches tricotées.

**22.** Structure tricotée selon l'une quelconque des revendications 1 à 21, incluant une pluralité desdits dispositifs de transducteur.

**23.** Système de détection comprenant une structure tricotée selon l'une quelconque des revendications précédentes; un moyen d'alimentation électrique pour appliquer un signal électrique sur le dispositif de transducteur tricoté; et un moyen de détection pour surveiller une caractéristique électrique dudit au moins un dispositif de transducteur tricoté.

**24.** Système selon la revendication 23, incluant une pluralité des dispositifs de transducteur tricotés, et dans lequel le moyen de détection est adapté pour dériver une information concernant l'orientation spatiale relative des dispositifs de transducteur.

**25.** Système selon la revendication 24, dans lequel le moyen de détection dérive une information concernant les orientations relatives des dispositifs de transducteur tricotés en comparant des caractéristiques électriques surveillées des dispositifs de transducteur tricotés.

**26.** Système selon l'une quelconque des revendications 23 à 25, dans lequel les caractéristiques électriques sont surveillées à une pluralité de fréquences.

27. Système selon la revendication 26, dans lequel les caractéristiques électriques sont surveillées à une pluralité de fréquences dans la plage de 1 à 1000 Hz.

28. Système selon la revendication 26 ou 27, dans lequel le moyen de détection produit un ECG à partir des caractéristiques électriques surveillées.

29. Vêtement comprenant une structure tricotée selon l'une quelconque des revendications 1 à 22.

30. Vêtement selon la revendication 29, comprenant une pluralité de couches tricotées.

31. Vêtement selon la revendication 30, dans lequel les couches tricotées sont formées en tant que structures tricotées d'un seul tenant.

32. Vêtement selon la revendication 30 ou 31, comprenant une première couche tricotée comportant au moins un dispositif de transducteur tricoté et dans lequel la seconde couche tricotée est tricotée avec des fibres électriquement conductrices qui jouent le rôle de voies de connexion pour le dispositif de transducteur tricoté.

33. Vêtement selon l'une quelconque des revendications 30 à 32, comprenant trois couches tricotées ou plus.

34. Vêtement selon la revendication 33, comprenant une première couche tricotée comportant un ou plusieurs dispositifs de transducteur tricotés; une seconde couche tricotée; et une troisième couche tricotée comportant un ou plusieurs dispositifs de transducteur tricotés.

35. Vêtement selon la revendication 34, dans lequel la seconde couche tricotée est adaptée pour filtrer des signaux électriques qui émanent de ou qui sont introduits dans un dispositif de transducteur tricoté situé dans la troisième couche tricotée.

36. Vêtement selon la revendication 35, dans lequel un dispositif de transducteur tricoté dans la troisième couche est un dispositif de transducteur tricoté inductif.

37. Vêtement sans couture selon l'une quelconque des revendications 29 à 36.

12    10

12

*Fig.1*

C

Conductive
elastomer →

Y   Direction of
courses

*Fig.2*

32          30

36

34

36

*Fig.3*

Fig.4

Fig.5(a)

Fig.5(b)

Fig.6

70

72

**_Fig.7_**

Amplitude in Ω

log10(f)   **_Fig.8 (a)_**

Phase in degrees

log10(f)   **_Fig.8 (b)_**

S3

β

S2

S1

α

**_Fig.9 (a)_**

**_Fig.9 (b)_**

100

102
104
106
108

*Fig.10*

110
112
114
116

*Fig.11*

120    122
X
124
122
120
X'

*Fig.12(a)*

X
122
122
120
124
122
X'

*Fig.12(b)*

130 →

132 134 136 138 140

**_Fig.13_**

142 144 146

Layer 1
Sensor Layer

Layer 2
Connecting Layer

Layer 3
Electrodes & Strain Gauge Layer

**_Fig.14(a)_**

144 148 144

146 142 142 146

**_Fig.14(b)_**

146 144 142

148

**_Fig.14(c)_**

150

back

mid

ont

152 154 156

**Fig. 15 (a)**

**Fig. 15 (b)**

Second welt
Vest rear layer.

Third welt Vest
middle layer.

First welt
Vest front layer.

^ Front of Vest ^

**Fig. 16**

Finish course

Tubular front

Tubular rear

Welt start

**Fig.17**

S4

S3

S2

S1

**Fig.18**

**Fig.19**

**Fig.20**

**Fig.21**

**Fig.22**

**Fig.23**

240

242

**Fig.24**

S2   **Fig.25**   S1

Fig.26

Fig.28

*Fig.27(a)*

*Fig.27(b)*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0240091 A
- WO 03094717 A
- US 6341504 B
- WO 0102052 A
- GB 2116725 A